# EUROPEAN PATENT APPLICATION

(11) **EP 2 730 923 A1**
(43) Date of publication of application: **14.05.2014**
(21) Application number: 12191981.5
(22) Date of filing: 09.11.2012
(51) Int. Cl.: G01N 33/68

(54) **NTproBNP and cTnT based therapy guidance in heart failure**

(71) Applicant: Roche Diagnostics GmbH, 68305 Mannheim (DE); F. Hoffmann-La Roche AG, 4070 Basel (CH)
(72) Inventor: Block, Dirk, 83673 Bichl (DE); Braz, Julian, Greenwood, IN 46143 (US); Brunner, Hans-Peter, 4142 Münchenstein (CH); Creeden, James, 4051 Basel (CH); Loyda, Hans-Jürgen, Carmel, IN 46033 (US); Wienhues-Thelen, Ursula-Henrike, 85152 Krailling (DE); Zaugg, Christian, 4310 Rheinfelden (CH)
(74) Representative: Dick, Alexander

(57) **Abstract**

The present invention relates to a method for guiding heart failure treatment in a subject suffering from heart failure. The method is based on the determination of the amount of a BNP-type peptide and a cardiac troponin in a sample from said subject. Further envisaged by the present invention are kits and devices adapted to carry out the present invention. The present invention also relates to a system for guiding heart failure treatment in a subject suffering from heart failure as disclosed herein and to reagents and kits used in performing the methods disclosed herein.

## Description

The present invention relates to a method for guiding heart failure treatment in a subject suffering from heart failure. The method is based on the determination of the amount of a BNP-type peptide and a cardiac troponin in a sample from said subject. Further envisaged by the present invention are kits and devices adapted to carry out the present invention. The present invention also relates to a system for guiding heart failure treatment in a subject suffering from heart failure as disclosed herein and to reagents and kits used in performing the methods disclosed herein.

An aim of modem medicine is to provide personalized or individualized treatment regimens. Those are treatment regimens which take into account a patient's individual needs or risks. Personalized or individual treatment regimens shall be even taken into account for measures where it is required to decide on potential treatment regimens.

Heart failure (HF) is a major and growing public health problem. It is estimated that approximately 5 million patients in the USA have HF, more than 500 000 patients are diagnosed with HF for the first time each year, and more than 250.000 patients in the US die each year of HF as a primary cause. Heart failure (HF) is one of the main causes of morbidity and mortality in developed countries. Because of aging of the population and greater longevity of patients with cardiovascular disease incidence and prevalence of HF are increasing.

Heart failure is a complex clinical syndrome that can result from any structural or functional cardiac disorder that impairs the ability of the ventricle to fill with or eject blood and to ensure the body's metabolic needs for supply with blood/oxygen. In such cases, the body tries to compensate lack of supply by structural changes of the myocardium (e.g. hypertrophy eventually leading to fibrosis, apoptosis, necrosis) and neurohumoral stimulation (activation of sympathetic nervous system and renin angiotensin aldosterone system) aiming at maintaining the required supply. HF is classified into various degrees of severity.

One classification is the so-called NYHA (New York Heart Association) classification. Heart failure patients are classified NYHA classes I, II, III and IV or American College of Cardiology and the American Heart Association (ACC/AHA) stages A, B, C, and D. Patients of NYHA Class I have no obvious symptoms of cardiovascular disease but already have objective evidence of functional impairment. Patients of NYHA class II have slight limitation of physical activity. Patients of NYHA class III show a marked limitation of physical activity. Patients of NYHA class IV are unable to carry out any physical activity without discomfort. They show symptoms of cardiac insufficiency at rest.

This functional classification is supplemented by the more recent classification by the American College of Cardiology and the American Heart Association (see J. Am. Coll. Cardiol. 2001;38;2101-2113, updated in 2005, see J. Am. Coll. Cardiol. 2005;46;el-e82). 4 stages A, B, C and D are defined. A patient having heart failure stage B, C or D has already experienced structural and functional changes to the heart. He will not be able to fully restore his health, and is in need of a therapeutical treatment.

WO2008/015254 discloses a GDF-15 detection based method of identifying a subject being susceptible to a therapy of heart failure, preferably a medicament based therapy using a medicament like an aldosterone antagonist including Spironolacton. Also a cardiac Troponin or NT-proBNP may be detected.

WO2010/007041 discloses a GDF-15, NT-proANP, NT-proBNP, and cardiac troponin detection based method of monitoring an apparently stable subject suffering from heart failure. Moreover it discloses a method of diagnosing and/or deciding which therapy/medication is to be applied in an apparently stable subject suffering from heart failure and undergoing a change in its physiological state.

WO2012/025355 discloses a Troponin and GDF-15 detection based method for therapy monitoring and therapy adaptation in a heart failure patient receiving administration of aldosterone antagonists like Spironolacton. The document further discloses that Troponin T, NT-proBNP and GDF15 detection allows monitoring or adaptation of therapy in heart failure patients.

Kubo et al. 2011 (Circulation J, 75, 919-926) discloses a BNP and Troponin based detection based risk prediction for clinical deterioration in patients suffering from hypertrophic cardiomyopathy. Many of the assessed patients suffered from heart failure. It is speculated that combined measurement of the two markers may be useful for monitoring patients with hypertrophic cardiomyopathy.

Fonarow et al. 2008 (Am J Cardiol, 101, 231-237) discloses a BNP and Troponin detection based mortality risk prediction in patients suffering from heart failure.

Mentz et al. 2011 (Circ J, 75(9):2031-7) discloses a NTproBNP detection based method for therapy guidance and monitoring in a heart failure patient, wherein the therapy is selected from treatment with diuretics, ACE inhibitor, beta blocker, spironolactone, nitrates or digoxin.

Böhm et al. 2011 (Clin Res Cardiol, 100:973-981) reviews NTproBNP detection based methods for therapy guidance and monitoring in a heart failure patient. It also mentions the possibility of combining BNP with troponin for guiding heart failure therapy.

Although available treatment options can reduce morbidity and mortality in patients with HF, the relative number of eligible patients receiving these treatments remains unsatisfactorily low (O'Donoghue M. & Braunwald E., Nat. Rev.Cardiol. 2010; 7: 13-20). Furthermore, in patients eligible for treatment, therapy has been primarily guided and adjusted by signs and symptoms of HF to maximal tolerability of drugs (e.g. by NYHA stages,ACC/AHA stages, or congestion scores). Measurement of natriuretic peptide markers, such as B-type natriuretic peptide (BNP), or its amino-terminal fragment N-terminal proB NP (NT-proBNP), has emerged as an important tool for the diagnosis and risk stratification of patients with HF. Additionally, there is emerging evidence that NT-proB NP is useful in guiding medical therapy in heart failure (Januzzi, Journal of Cardiac Failure, 2011; 17: 622-625), whereas guiding therapy comprises serial measurements of NT-proBNP to identify and monitor patients that are at increased risk and that could benefit from intensification of therapy, and to monitor the effect of therapy intensification on NT-proBNP levels

However, NT-proBNP guided HF therapy does not identify all patients at risk of HF decompensation and of adverse events. Consequently, some patients remain at risk even though they show favorable response to therapy with regards to their NTproBNP levels.

The technical problem underlying the present invention can be seen as the provision of means and methods that allow to guide heart failure therapy more reliably.

The technical problem is solved by the embodiments characterized in the claims and herein below.

Advantageously, it was shown in the context of the studies that the combination of NTproBNP with a cardiac Troponin can be reliably used for monitoring purposes and as a guide for therapy in addition to current standard-of-care to adjust and titrate therapy in HF patients. Specifically, addition of Troponin measurements to NT-proBNP or BNP together with current standard-of-care allows for further risk stratification and monitoring HF patients who may be in need for more intensified therapy and closer observation.

Accordingly, the present invention relates to a method for guiding heart failure treatment in a subject suffering from heart failure, said method comprising the steps of
a) determining the amount of a BNP-type peptide and of a cardiac Troponin in a sample from the subject, and
b) comparing the amount of the BNP-type peptide to a reference amount for the BNP-type peptide, and the amount of the cardiac Troponin to a reference amount for the cardiac Troponin, whereby heart failure treatment is guided.

Preferably, a heart failure treatment is guided by carrying out the further step c) of guiding heart failure treatment, based on the results of the comparison carried out in step b).

The method of the present invention, preferably, is an ex vivo or in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or assessment based on said comparison in step (b).

Accordingly, the present invention also preferably relates to a system for guiding heart failure treatment in a subj ect suffering from heart failure, comprising
a) an analyzer unit configured to contact, in vitro, a portion of a sample from the subject with a ligands comprising specific binding affinity for a BNP-type peptide and a cardiac Troponin,
b) an analyzer unit configured to detect a signal from the portion of the sample from the subject contacted with the ligand,
c) a computing device having a processor and in operable communication with said analysis units, and
d) a non-transient machine readable media including a plurality of instruction executable by a the processor, the instructions, when executed calculate the amounts of the markers, and compare the amounts of the markers with reference amounts, thereby guiding heart failure therapy.

The phrase "guiding heart failure treatment" as used herein, preferably, means assessing whether heart failure treatment of a subject suffering from heart failure has to be intensified (and, preferably, also monitored), or not. Accordingly, by carrying out the method of the present invention, a subject can be identified who is eligible to an intensification of heart failure treatment. Accordingly, the present invention also envisages a method of identifying a subject who is eligible to an intensification of heart failure treatment. Preferably, a subject who is eligible to an intensification of heart failure treatment will benefit from said intensification. In particular, a subject benefits from the intensification, if the risk of mortality of said subject and/or the risk of hospitalization of said subject, in particular within a window period of 18 months or 3 years, is reduced by said intensification. A subject who does not require intensification of said therapy preferably does not benefit from the intensification. In this case, e.g. the adverse side effects caused by said intensification may outweigh the benefits of the intensification.

As described herein below in more detail, a subject who is eligible to intensification of heart failure treatment, shall be also monitored at short intervals, whereas a subject who is not eligible to intensification of heart failure treatment (i.e. who does not require intensification of heart failure treatment) shall be monitored at long intervals. Therefore, in addition to the decision whether heart failure treatment shall be intensified or not, it can be assessed whether the subject shall be monitored at short intervals or long intervals.

As will be understood by those skilled in the art, the assessment made by the method of the present invention is usually not intended to be correct for 100% of the subjects to be diagnosed. The term, however, requires that the assessment is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "subject" as used herein in the context with the aforementioned method relates to animals, preferably mammals, and, more preferably, humans. It is envisaged in the context of the present invention, that the subject suffers from heart failure (HF), in particular from chronic heart failure.

The term "heart failure" as used herein relates to an impaired systolic and/or diastolic function of the heart being accompanied by overt signs of heart failure as known to the person skilled in the art. Preferably, heart failure referred to herein is also chronic heart failure. Heart failure according to the present invention includes overt and/or advanced heart failure. In overt heart failure, the subject shows symptoms of heart failure as known to the person skilled in the art.

HF can be classified into various degrees of severity.

According to the NYHA (New York Heart Association) classification, heart failure patients are classified as belonging to NYHA classes I, II, III and IV. A patient having heart failure has already experienced structural and functional changes to his pericardium, myocardium, coronary circulation or cardiac valves. He will not be able to fully restore his health, and is in need of a therapeutical treatment. Patients of NYHA Class I have no obvious symptoms of cardiovascular disease but already have objective evidence of functional impairment. Patients of NYHA class II have slight limitation of physical activity. Patients of NYHA class III show a marked limitation of physical activity. Patients of NYHA class IV are unable to carry out any physical activity without discomfort. They show symptoms of cardiac insufficiency at rest.

This functional classification is supplemented by the more recent classification by the American College of Cardiology and the American Heart Association (see J. Am. Coll. Cardiol. 2001;38;2101-2113, updated in 2005, see J. Am. Coll. Cardiol. 2005;46;el-e82). 4 stages A, B, C and D are defined. Stages A and B are not HF but are considered to help identify patients early before developing "truly" HF. Stages A and B patients are best defined as those with risk factors for the development of HF. For example, patients with coronary artery disease, hypertension, or diabetes mellitus who do not yet demonstrate impaired left ventricular (LV) function, hypertrophy, or geometric chamber distortion would be considered stage A, whereas patients who are asymptomatic but demonstrate LV hypertrophy and/or impaired LV function would be designated as stage B. Stage C then denotes patients with current or past symptoms of HF associated with underlying structural heart disease (the bulk of patients with HF), and stage D designates patients with truly refractory HF.

As used herein, the term "heart failure", in particular, refers to stages C and D of the ACC/AHA classification referred to above. In these stages, the subject shows typical symptoms of heart failure. Accordingly, a subject who suffers from heart failure suffers from heart failure stage B, C or D according to the ACC/AHA classification. Also preferably, the subject may be classified as to belong to NYHA class II, III or IV.

Preferably, the heart failure is due to impaired systolic function. Accordingly, it is, in particular, envisaged that the subject suffers from systolic heart failure. Preferably, the subject has a left ventricular ejection fraction (LVEF) of less 50 %, more preferably, of less than 45%, and most preferably, of less than 40%.

It is further envisaged that the subject did not experience worsening of heart failure signs and/or symptoms prior to obtaining the sample to be tested. Preferably, the subject did not experience worsening of heart failure signs and/or symptoms within a period of three months prior to obtaining the sample. Preferably, the subject did not experience worsening of heart failure symptoms within a period of six months prior to obtaining the sample. More preferably, the subject did not experience worsening of heart failure symptoms within a period of three months prior to obtaining the sample. Advantageously, it has been shown in the context of the present invention, that the combined determination of a cardiac Troponin and of BNP-type peptide also allows for identify subjects which require an intensification of heart failure therapy, if the subject did not experience worsening of heart failure symptoms before a carrying out the method of the present invention (i.e. before obtaining the sample to be tested in the context of the method of the present invention).

Signs and symptoms of heart failure are well known in the art. Preferably, the signs and symptoms of heart failure are those as described for subjects of NYHA classes II, III and IV. Preferably, the signs and/or symptoms are shortness of breath (Dyspnea), exercise intolerance, fatigue, edema, weight gain / ascites, tachycardia, and cough.

Further, it is envisaged that the subject in the context of the present invention does not have impaired renal function. Preferably, the subject shall not suffer from renal failure, in particular the subject shall not suffer from acute, chronic and/or end stage renal failure. Further, the subject, preferably, shall not suffer from renal hypertension. How to assess whether a subject exhibits impaired renal function is well known in the art. Renal disorders can be diagnosed by any means known and deemed appropriate. Particularly, renal function can be assessed by means of the glomerular filtration rate (GFR). For example, the GFR may be calculated by the Cockgroft-Gault or the MDRD formula (Levey 1999, Annals of Internal Medicine, 461-470). GFR is the volume of fluid filtered from the renal glomerular capillaries into the Bowman's capsule per unit time. Clinically, this is often used to determine renal function. All calculations derived from formulas such as the Cockgroft Gault formula of the MDRD formula deliver estimates and not the "real" GFR) by injecting inulin into the plasma. Since inulin is not reabsorbed by the kidney after glomerular filtration, its rate of excretion is directly proportional to the rate of filtration of water and solutes across the glomerular filter. In clinical practice however, creatinine clearance is used to measure GFR. Creatinine is an endogenous molecule, synthesized in the body, which is freely filtered by the glomerulus (but also secreted by the renal tubules in very small amounts). Creatinine clearance (CrCl) is therefore a close approximation of the GFR. The GFR is typically recorded in milliliters per minute (mL/min). The normal range of GFR for males is 97 to 137 mL/min, the normal range of GFR for females is 88 to 128 ml/min. Thus, it is particularly contemplated that the GFR of a subject who does not exhibit impaired renal function is within this range. Moreover, said subject preferably, has a blood creatinine level (in particular a serum creatinine level) of lower than 0.9 mg/dl, more preferably of lower than 1.1 mg/dl and most preferably of lower than 1.3 mg/dl.

Moreover, it is further envisaged that the subject does not suffer from ACS (acute coronary syndrome). The term "ACS" as used herein includes STEMI (ST-elevation myocardial infarction); NSTEMI (non ST-elevation myocardial infarction) and unstable angina pectoris. It is further envisaged that the subject to be tested does not have a history of ACS. In particular, the subject shall not have suffered from ACS within one month prior to carrying out the method of the present invention (to be more precise, within one month prior to obtaining the sample).

It is envisaged that the subject suffering from heart failure shall be treated for heart failure. Thus, the subject shall receive heart failure treatment.

The term "heart failure treatment" (herein also referred to as "heart failure therapy") as used herein, preferably, refers to any treatment that allows to treat heart failure. Preferably, the term encompasses life style changes, diet regimen, interventions on the body as well as administration of appropriate medicaments, use of devices and/or organ transplants for the treatment of the subject suffering from heart failure.

Life style changes include smoking cessation, moderation of alcohol consumption, increased physical activity, weight loss, sodium (salt) restriction, weight management and healthy eating, daily fish oil, salt restriction.

Preferred devices to be applied are pacemakers and resynchronisation devices, defibrillator, intra-aortic balloon pumps, and ventricular assist devices.

In a particularly preferred embodiment, the heart failure treatment encompasses administration of medicaments. Medicaments suitable for the treatment of heart failure are well known in the art, see e.g. Heart Disease, 2008, 8th Edition, Eds. Braunwald, Elsevier Sounders, chapter 24 or the ESC Guidelines for the diagnosis and treatment of acute and chronic heart failure (European Heart Journal (2008) 29, 2388-2442)

Preferably, the heart failure treatment includes administration of at least one medicament selected from the group consisting of angiotensin converting enzyme inhibitors (ACE inhibitors), angiotensin II receptor blockers (frequently also referred to as angiotensin II receptor antagonists), beta adrenergic blockers (herein also referred to as beta blockers), diuretics, aldosterone antagonists, adrenergic agonists, positive inotropic agents, and calcium antagonists, hydralazine, nitrates, aspirin. It is particularly preferred that the medicament is an angiotensin converting enzyme inhibitor, an angiotensin II receptor blockers, a beta blocker and/or an aldosterone blocking agent.

Preferred ACE-inhibitors include benazepril, captopril, cilazapril, enalapril, fosinopril, lisinopril, moexipril, perindopril, quinapril, ramipril, spirapril, and trandolapril. A particularly preferred inhibitor is enalapril.

Preferred beta blockers include cebutolol, alprenolol, atenolol, betaxolol, bisoprolol, bupranolol, carazolol, carteolol, carvedilol, celiprolol, metipranolol, metoprolol, nadolol, nebivolol, oxprenolol, penbutolol, pindolol, propanolol, sotalol, tanilolol, and timolol. A particularly preferred beta blocker is atenolol, bisoprolol, carvedilol, or metoprol.

Preferred angiotensin II receptor antagonists are Losartan, Valsartan, Irbesartan, Candesartan, Telmisartan, and Eprosartan. A particularly preferred antagonist is Losartan or Valsartan.

Preferred diuretics are loop diuretics, thiazide and thiazide-like diuretics, K-sparing diuretics, mineralocorticoid receptor antagonists, and vasopressin antagonists.

Preferred aldosterone antagonists are Eplerone, Spironolactone, Canrenone, Mexrenone, Prorenone; and statines, in particular Atorvastatin, Fluvastatin, Lovastatin, Pravastatin, Rosuvastatin, and Simvastatin. A particularly preferred antagonist is Spironolactone.

Preferred positive inotropic agents are digoxin and digitoxin.

Preferred calcium antagonists are dihydropyridines, verapamil, and diltiazem.

Preferred adrenergic agonists are dobutamine, dopamine, epinephrine, isoprotenerol, norepinephrine, and phenylephrine.

The heart failure treatment to be guided can be any treatment as set forth herein above. In a preferred embodiment, however, the treatment to be guided includes administration of at least one medicament as set forth above. In an even more preferred embodiment, the heart failure treatment comprises administration of at least one medicament selected from the group consisting of an angiotensin converting enzyme inhibitor, an angiotensin II receptor blocker, and a beta blocker. Most preferably, the heart failure treatment to be guided comprises the combined administration of a beta blocker and an ACE inhibitor.

In accordance with the method of the present invention, it shall be assessed whether heart failure treatment of the subject to be tested shall be intensified, or not. Preferably, the intensification of heart failure treatment comprises at least one of the following:
● increasing the dosage of a previously administered medicament or of previously administered medicaments,
● the administration of a further medicament (or medicaments), in particular the administration of a further medicament (or medicaments) having a different mode of action that the previously administered medicaments,
● device therapy, in particular use of pacemaker devices, cardiac resynchonization therapy (CRT), implantable defibrillator devices (ICD) or left ventricular assist devices (LVAD) life style changes, and
● combinations thereof.

Preferably, the intensification comprises increasing the dosage of a previously administered medicament or of previously administered medicaments, in particular increasing the dosage of selected from the group consisting of a diuretic, an angiotensin converting enzyme inhibitor, an angiotensin II receptor blocker, an aldosterone antagonist, and a beta blocker. How to increase the dosage, it well known in the art, and, e.g., may be derived from the guidelines. Preferably, the dosing of these medicaments may be increased until the maximally recommended therapeutic dose or until the maximally tolerated dose, whatever is reached first.

Also preferably, the intensification comprises the administration of a further medicament (or medicaments), in particular the administration of a further medicament (or medicaments) having a different mode of action than the previously administered medicaments, or the application of further devices (i.e. of medicaments/devices that were not administered/used prior to carrying out the method of the present invention). Preferred further medicaments include hydralazin, nitrates, inotropic agents, adrenergic agents. Preferred devices include pacemaker devices, cardiac resynchronization therapy (CRT), and implantable defibrillator devices (ICD).

Also, the intensification of heart failure treatment may further encompass monitoring the subject at short intervals. Accordingly, by carrying out the method of the present invention a subject can be identified who requires closer monitoring, in particular with respect to the heart failure therapy (and, thus, closer observation). With "closer monitoring" it is, preferably, meant that biomarkers as referred herein, i.e. the cardiac Troponin and the BNP-type peptide are determined in at least one further sample obtained from the subject after a short interval after the sample referred to in step a) of the method of the present invention. Preferred short intervals are mentioned herein below.

A subject who does not require intensification of heart failure treatment, preferably, can continue the heart failure treatment without changing the treatment regimen. Thus, it is not necessary to adapt the dosage of the administered medicament(s) and/or to change the medicaments.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

The term "cardiac Troponin" refers to all Troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac Troponin refers to Troponin T and/or Troponin I, and, most preferably, to Troponin T. It is to be understood that isoforms of Troponins may be determined in the method of the present invention together, i.e. simultaneously or sequentially, or individually, i.e. without determining the other isoform at all. Amino acid sequences for human Troponin T and human Troponin I are disclosed in Anderson, loc cit and Ferrieres 1998, Clinical Chemistry, 44: 487-493.

The term "cardiac Troponin" encompasses also variants of the aforementioned specific Troponins, i.e., preferably, of Troponin I, and more preferably, of Troponin T. Such variants have at least the same essential biological and immunological properties as the specific cardiac Troponins. In particular, they share the same essential biological and immunological properties if they are detectable by the same specific assays referred to in this specification, e.g., by ELISA Assays using polyclonal or monoclonal antibodies specifically recognizing the said cardiac Troponins. Moreover, it is to be understood that a variant as referred to in accordance with the present invention shall have an amino acid sequence which differs due to at least one amino acid substitution, deletion and/or addition wherein the amino acid sequence of the variant is still, preferably, at least about 50%, at least about 60%, at least about 70%, at least about 80%, at least about 85%, at least about 90%, at least about 92%, at least about 95%, at least about 97%, at least about 98%, or at least about 99% identical with the amino sequence of the specific Troponin (in particular over the entire length). Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad. Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants may be allelic variants or any other species specific homologs, paralogs, or orthologs. Moreover, the variants referred to herein include fragments of the specific cardiac Troponins or the aforementioned types of variants as long as these fragments have the essential immunological and biological properties as referred to above. Preferably, the cardiac troponin variants have immunological properties (i.e. epitope composition) comparable to those of human troponin T or troponin I. Thus, the variants shall be recognizable by the aforementioned means or ligands used for determination of the concentration of the cardiac troponins. Thus, the variants shall be recognizable by the aforementioned means or ligands used for determination of the concentration of the cardiac troponins. Such fragments may be, e.g., degradation products of the Troponins. Further included are variants which differ due to posttranslational modifications such as phosphorylation or myristylation. Preferably the biological property of troponin I and its variant is the ability to inhibit actomyosin ATPase or to inhibit angiogenesis in vivo and in vitro, which may e.g. be detected based on the assay described by Moses et al. 1999 PNAS USA 96 (6): 2645-2650). Preferably the biological property of troponin T and its variant is the ability to form a complex with troponin C and I, to bind calcium ions or to bind to tropomyosin, preferably if present as a complex of troponin C, I and T or a complex formed by troponin C, troponin I and a variant of troponin T. It is known that low concentrations of circulating cardiac troponin may be detected in subjects at various conditions, but further studies are required to understand their respective role and rate (Masson et al., Curr Heart Fail Rep (2010) 7:15-21).

Preferably, the cardiac Troponin is Troponin T, in particular human Troponin T. Preferably, the amount of Troponin T is determined by using an high sensitive Troponin assays as described in the Examples, or in WO2012/025355.

As used herein, the term "BNP-type peptides" comprise pre-proBNP, proBNP, NT-proBNP, and BNP. The pre-pro peptide (134 amino acids in the case ofpre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP). Preferably, BNP-type peptides according to the present invention are NT-proBNP, BNP, and variants thereof. BNP is the active hormone and has a shorter half-life than the respective inactive counterpart NT-proBNP. BNP is metabolized in the blood, whereas NT-proBNP circulates in the blood as an intact molecule and as such is eliminated renally. The in-vivo half-life of NT-proBNP is 120 min longer than that of BNP, which is 20 min (Smith 2000, J Endocrinol. 167: 239-46.). Preanalytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measurement of the active or the inactive forms of the natriuretic peptide can be advantageous. The most preferred natriuretic peptides according to the present invention are NT-proBNP or variants thereof. As briefly discussed above, the human NT-proBNP, as referred to in accordance with the present invention, is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein. The NT-proBNP referred to in accordance with the present invention further encompasses allelic and other variants of said specific sequence for human NT-proBNP discussed above. Specifically, envisaged are variant polypeptides which are on the amino acid level preferably, at least 50%, 60%, 70%, 80%, 85%, 90%, 92%, 95%, 97%, 98%, or 99% identical to human NT-proBNP, preferably over the entire length of human NT-proBNP. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad. Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of human NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Lab Invest 230:177-181), Yeo et al. (Yeo 2003, Clinica Chimica Acta 338:107-115). Variants also include posttranslationally modified peptides such as glycosylated peptides. Further, a variant in accordance with the present invention is also a peptide or polypeptide which has been modified after collection of the sample, for example by covalent or non-covalent attachment of a label, particularly a radioactive or fluorescent label, to the peptide.

Determining the amount of a peptide or polypeptide referred to in this specification relates to measuring the amount or concentration, preferably, semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay and methods which may utilize labeled molecules in various sandwich, competition, or other assay formats. Such assays are, preferably, based on detection agents such as antibodies which specifically recognize the peptide or polypeptide to be determined. The detection agents shall be either directly or indirectly capable of generating a signal indicating the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on ElecsysTM analyzers), CBA (an enzymatic Cobalt Binding Assay, available for example on Roche-HitachiTM analyzers), and latex agglutination assays (available for example on Roche-HitachiTM analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) (optionally) removing non-bound ligand, (c) measuring the amount of bound ligand.

According to a preferred embodiment, said steps of contacting, removing and measuring may be performed by an analyzer unit of the system disclosed herein. According to some embodiments, said steps may be performed by a single analyzer unit of said system or by more than one analyzer unit in operable communication with each other. For example, according to a specific embodiment, said system disclosed herein may include a first analyzer unit for performing said steps of contacting and removing and a second analyzer unit, operably connected to said first analyzer unit by a transport unit (for example, a robotic arm), which performs said step of measuring.

The bound ligand, in particular the ligand or the ligand/peptide complex, will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)2 fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semiquantitative or quantitative. Further suitable techniques for the determination of a polypeptide or peptide are described in the following.

First, binding of a ligand may be measured directly, e.g. by NMR or surface plasmon resonance. Measurement of the binding of a ligand, according to preferred embodiments, is performed by an analyzer unit of a system disclosed herein. Thereafter, an amount of the measured binding may be calculated by a computing device of a system disclosed herein. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include 35S, 125I, 32P, 33P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations. According to preferred embodiments of the subject invention, the determination of an "amount" is performed by the disclosed system, whereby a computing device determines the "amount" based on contacting and measuring steps performed by one or more analyzer units of said system.

The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. Thus, the comparison referred to in step (b) of the method of the present invention may be carried out by a computing device (e.g., of a system disclosed herein). The value of the amount and the reference can be, e.g., compared to each other and the said comparison can be automatically carried out by a computer program executing an algorithm for the comparison. The computer program carrying out the said evaluation will provide the desired assessment in a suitable output format. For a computer assisted comparison, the value of the determined amount may be compared to values corresponding to suitable references which are stored in a database by a computer program. The computer program may further evaluate the result of the comparison, i.e. automatically provide the desired assessment in a suitable output format. For a computer assisted comparison, the value of the determined amount may be compared to values corresponding to suitable references which are stored in a database by a computer program. The computer program may further evaluate the result of the comparison, i.e. automatically provides the desired assessment in a suitable output format. The said result may, preferably, serve as an aid for guiding heart failure treatment in the subj ect.

Based on the comparison of the amount determined in step a) and the reference amount, it shall be possible to assess whether the subject can continue heart failure treatment or whether heart failure treatment has to be intensified. For example, a result of a comparison may be given as raw data (absolute or relative amounts), and in some cases as an indicator in the form of a word, phrase, symbol, or numerical value which may be indicative of a particular assessment. Therefore, the reference amount is to be chosen so that either a difference or an identity in the compared amounts allows identifying those test subjects which belong into the group of subjects which can continue heart failure treatment, or those test subjects in which heart failure treatment has to be intensified. The assessment may be provided by the computing device of a system disclosed herein based on said comparison of the calculated "amount" to a reference or a threshold. For example, a computing device of a system may provide an indicator, in the form of a word, symbol, or numerical value which is indicative for the assessment. Differences in the amounts, i.e. increases or decreases, as used herein, preferably, are differences which are statistically significant. Whether a difference is statistically significant can be determined by the statistical techniques referred to elsewhere herein. Similarly, an identity in the amounts encompasses identical amounts and those differences in the amounts which are not statistically significant and which are within the standard deviations for a measured parameter.

The term "reference amount" as used herein refers to an amount which allows for allocation of a subject into either (i) the group of subjects who can continue heart failure treatment or (ii) the group of subjects in which heart failure treatment has to be intensified. The reference amount applicable for an individual subject may vary depending on various physiological parameters such as age, gender, or subpopulation, as well as on the means used for the determination of the polypeptide or peptide referred to herein. A suitable reference amount may be determined from a reference sample to be analyzed together, i.e. simultaneously or subsequently, with the test sample.

Preferably, the following diagnostic algorithm is applied if one biomarker is determined, i.e. a cardiac Troponin or a BNP-type peptide.

In principle, an amount of the biomarker in the sample from the test subject which is increased as compared to the reference amount is indicates that the heart failure treatment shall be intensified, wherein an amount of the biomarker in the sample from the test subject which is decreased as compared to the reference amount indicate(s) that the heart failure treatment shall not be intensified.

Preferably, the following diagnostic algorithm is applied if two biomarkers are determined, i.e. a cardiac Troponin and a BNP-type peptide.

Preferably
● an amount of the BNP-type peptide in the sample from the test subject and an amount of the cardiac Troponin which are both increased as compared to the reference amount (for the BNP-type peptide and the cardiac Troponin, respectively) indicates that the heart failure treatment shall be intensified
● an amount of the BNP-type peptide in the sample from the test subject which is increased as compared to the reference amount for the BNP-type peptide, or an amount of the cardiac Troponin which is increased as compared to the reference amount for the cardiac Troponin indicates that the heart failure treatment shall be intensified
● an amount of the BNP-type peptide in the sample from the test subject and an amount of the cardiac Troponin which are both decreased as compared to the reference amount (for the BNP-type peptide and the cardiac Troponin, respectively) indicates that the heart failure treatment shall not be intensified.

Preferred reference amounts are indicated herein below.

Reference amounts can, in principle, be calculated for a cohort of subjects as specified above based on the average or mean values for a given biomarker by applying standard methods of statistics. Further, it is envisaged that the reference amount (the reference amounts) is (are) from (i) a subject or group of subjects known to require an intensification of heart failure therapy and/or (ii) a subject or group of subjects known not to require an intensification of heart failure therapy.

Preferably, the following applies as diagnostic algorithm:
Preferably, the reference amounts for the biomarkers referred to herein, i.e. for a cardiac Troponin and/a BNP-type peptide are derived from a subject or group of subjects known to require an intensification of heart failure therapy, wherein
   ● amounts of both markers in the test sample which are increased as compared to the reference amounts for both markers, and/or amounts of both markers in the test sample which are essentially the same as compared to the reference amounts for both markers, or
   ● an amount of a cardiac Troponin or an amount of the BNP-type peptide in the test sample which is increased or which is essentially the same as compared to the reference amount, is indicative for a subject who requires an intensification of heart failure therapy.

Additionally or alternatively, the reference amounts for the biomarkers referred to herein, i.e. for a cardiac Troponin and/a BNP-type peptide are derived from a subject or group of subjects known not to require an intensification of heart failure therapy, and thus from a subject or group of subjects who can continue heart failure treatment without altering the treatment region, wherein amounts of both markers in the test sample which are decreased as compared to the reference amounts, and/or amounts of both markers in the test sample which are essentially the same as compared to the reference amounts for both markers, is (are) indicative for a subject who does not require an intensification of heart failure therapy, and, thus, who can continue heart failure therapy (in particular without changing the treatment regimen).

In a preferred embodiment of the method of the present invention, the method does not encompass the determination of the amount of GDF-15 (Growth Differentiation Factor 15). It has been shown in the context to the present invention that heart failure therapy can be reliably guided based on the determination of a cardiac Troponin and a BNP-type peptide, even if GDF-15 is not determined.

Preferred reference amounts to be applied in the context of the method of the present invention are those described in the Examples. Preferred reference are about 100 pg/ml for BNP, about 1000 pg/ml for NT-proBNP, about 23.5 pg/ml for Troponin T, about 50 pg/ml for Troponin I.

Preferably, the term "about" as used herein encompasses a range of + and - 20%, more preferably a range of + and - 10%, even more preferably a range of + and - 5%, and most preferably a range of + and - 2%, relative to the specific amount, e.g., indication of a an amount of "about 100" is meant to encompass an amount within a range from 80 to 120. Also, the term "about" refers to the exact amount.

Depending on the results of the method of the present invention, i.e. depending whether the subject requires intensification of heart failure treatment, or not, further steps may be carried out. As it was already set forth above, decisions can be made with respect to the further monitoring of the subject. Preferably, a subject who requires an intensification of heart failure therapy also requires a monitoring at short intervals, whereas a subject who does not require said intensification does also not require monitoring at short intervals (and, thus, may be monitored at long intervals).

Preferably, the subject, i.e. the heart failure treatment of said subject, is monitored by determining the amounts of the biomarkers as referred to herein in at least one further sample from said subject. Depending on the results of the method of the present invention (i.e. of steps a) and b)), the further sample, is preferably, obtained after a short interval or after a long interval after the sample as set forth in step a).

Accordingly, the method of the present invention, preferably, comprises the further steps of
c) determining the amount of a BNP-type peptide and of a cardiac Troponin in a further sample from the subject, and
d) comparing the amount of the BNP-type peptide in said further sample to a reference amount for the BNP-type peptide, and the amount of the cardiac Troponin in said further sample to a reference amount for the cardiac Troponin.

The further sample referred to in step c) shall have been obtained after the sample referred to in step a).

The reference amounts and the diagnostic algorithms with respect to step d) are, preferably, the same as for step b).

By carrying out steps c) and d), it is assessed whether the subject suffering from heart failure requires an intensification / a further intensification of heart failure treatment.

Preferably, steps c) and d) are carried out, if the subject requires an intensification of heart failure treatment (according to steps a) and b) of the method of the present invention). In this case, the further steps c) and d) allow for assessing whether the subject requires a further intensification of heart failure treatment.

However, steps c) and d) may be also carried out if the subject does not require an intensification of heart failure treatment (according to steps a) and b) of the method of the present invention). In this case, the further steps c) and d) allow for assessing whether the subject requires an intensification of heart failure treatment.

If - according to steps a) and b) of method of the present invention - the subject requires an intensification of heart failure treatment, preferably, the following applies: Preferably, said further sample referred to above has been obtained between two weeks and four months after the sample referred to in step a). More preferably, said further sample has been obtained between four weeks and three months after the sample referred to in step a). Even more preferably, said further sample has been obtained between two weeks and two months after the sample referred to in step a). Most preferably, said further sample has been obtained about six weeks after the sample referred to in step a). The aforementioned periods/intervals are considered as short intervals.

Preferably, steps c) and d) are repeated with the intervals referred to above until the amounts of the cardiac Troponin and the BNP-type peptide are lower than the reference amounts. This, preferably, indicates that the subject does not require heart failure intensification, i.e. does not require further heart failure intensification. In this case, steps c) and d) are repeated with the intervals referred to in the next paragraph.

If - according to steps a) and b) of the method of the present invention - the subject does not require an intensification of heart failure treatment, preferably, the following applies: Preferably, said further sample referred to above has been obtained between three months and twelve months after the sample referred to in step a). More preferably, said further sample has been obtained between four months and ten months after the sample referred to in step a). Even more preferably, said further sample has been obtained between five months and nine months after the sample referred to in step a). Most preferably, said further sample has been obtained between six to eight months after the sample referred to in step a). The aforementioned periods/intervals are considered as long intervals.

In an aspect of the invention, a method for guiding heart failure treatment in a subject suffering from heart failure, is contemplated, said method comprising:
a) determining the amount of a BNP-type peptide and of a cardiac Troponin by (i) bringing the sample into contact with a detection agent that specifically binds to said BNP-type peptide and with a detection agent that specifically binds to said cardiac troponin for a time sufficient to allow for the formation of a complex of the said detection agent and the marker from the sample, (ii) measuring the amounts of the formed complexes, wherein the said amounts of the formed complexes are proportional to the amount of the marker present in the sample, and (iii) transforming the amounts of the formed complexes into amounts of the markers reflecting the amounts of the markers present in the sample;
b) comparing said amounts to reference amounts; and
c) establishing an aid for guiding heart failure treatment in a subject suffering from heart failure based on the result of the comparison made in step b).

In another aspect of the invention, a system for establishing an aid for guiding heart failure treatment in a subject suffering from heart failure, is contemplated, comprising:
a) an analyzer unit configured to bringing the sample into contact with a detection agent that specifically binds to said BNP-type peptide and with a detection agent that specifically binds to said cardiac troponin for a time sufficient to allow for the formation of a complexes of the said detection agent and the marker from the sample,
b) an analyzer unit configured to measure the amounts of the formed complexes, wherein the said amounts of the formed complexes are proportional to the amounts of the markers present in the sample,
c) a computing device having a processor and in operable communication with said analysis units, and
d) a non-transient machine readable media including a plurality of instructions executable by the processor, the instructions, when executed transform the amount of the formed complex into an amount of the markers reflecting the amount of the markers in the sample, compare said amounts to reference amounts, and establish an aid for guiding heart failure treatment in a subject suffering from heart failure based on the result of said comparison to said reference.

A suitable detection agent may be, in an aspect, an antibody which is specifically binds to the at least one marker, i.e. a detection agent which binds to a BNP-type peptide, or a detection agent which binds to a cardiac troponin, in a sample from a subject to be investigated by the method of the invention. Another detection agent that can be applied, in an aspect, may be an aptamere which specifically binds to the marker. In yet an aspect the, sample is removed from the complex formed between the detection agent and the marker prior to the measurement of the amount of formed complex. Accordingly, in an aspect, the detection agent may be immobilized on a solid support. In yet an aspect, the sample can be removed from the formed complex on the solid support by applying a washing solution. The formed complex shall be proportional to the amount of the at least one marker present in the sample. It will be understood that the specificity and/or sensitivity of the detection agent to be applied defines the degree of proportion of at least one marker comprised in the sample which is capable of being specifically bound. Further details on how the determination can be carried out are also found elsewhere herein. The amount of formed complex shall be transformed into an amount of at least one marker reflecting the amount indeed present in the sample. Such an amount, in an aspect, may be essentially the amount present in the sample or may be, in another aspect, an amount which is a certain proportion thereof due to the relationship between the formed complex and the amount present in the original sample.

In yet an aspect of the aforementioned method, step a) may be carried out by an analyzer unit, in an aspect, an analyzer unit as defined elsewhere herein.

In an aspect of the method of the invention, the amounts determined in step a) are compared to reference amounts. In an aspect, the reference amounts are reference amounts as defined elsewhere herein. In yet another aspect, the reference takes into account the proportional relationship between the measured amount of complex and the amount present in the original sample. Thus, the references applied in an aspect of the method of the invention are artificial references which are adopted to reflect the limitations of the detection agent that has been used. In another aspect, said relationship can be also taken into account when carrying out the comparison, e.g., by including a normalization and/or correction calculation step for the determined amount prior to actually comparing the value of the determined amount and the reference. Again, the normalization and/or correction calculation step for the determined amount adopts the comparison step such that the limitations of the detection agent that has been used are reflected properly. In an aspect, the comparison is carried out automatically, e.g., assisted by a computer system or the like.

The aid for guiding heart failure treatment in a subject suffering from heart failure is established based on the comparison carried out in step b) by allocating the subj ect either into a group of subjects being in need of therapy intensification, or not, as set forth herein elsewhere. As discussed elsewhere herein already, the allocation of the investigated subject must not be correct in 100% of the investigated cases. Moreover, the groups of subjects into which the investigated subject is allocated are artificial groups in that they are established based on statistical considerations, i.e. a certain preselected degree of likelihood based on which the method of the invention shall operate. In an aspect of the invention, the aid for optimizing a risk assessment is established automatically, e.g., assisted by a computing device or the like, as described and disclosed herein.

In an aspect of the method of the invention, said method further comprises a step of recommending and/or managing the subject according to the result established in step c) as set forth elsewhere herein in detail, and/or adapting intensiveness of disease monitoring.

In an aspect of the aforementioned method, steps b) and/or c) are carried out by one or more analyzer units as set forth elsewhere herein.

The present invention also relates to the use of i) of a BNP-type peptide and of a cardiac Troponin or ii) of a detection agent which specifically binds to a BNP-type peptide, and/or of a detection agent which specifically binds to a cardiac Troponin, in a sample from a subject suffering from heart failure for guiding heart failure treatment.

The term "detection agent" as used herein refers to an agent that is capable of specifically recognizing and binding to the biomarker polypeptide(s) present in a sample. Moreover, the said agent shall allow for direct or indirect detection of the complex formed by the said agent and the biomarker. Direct detection can be achieved by including into the agent a detectable label. Indirect labelling may be achieved by a further agent that specifically binds to the complex comprising the biomarker and the detection agent wherein the said further agent is than capable of generating a detectable signal. Suitable compounds which can be used as detection agents are well known in the art. Preferably, the detection agent is an antibody or aptamere which specifically binds to the biomarker. The term "antibody" has been described elsewhere herein.

According to a preferred embodiment of the present invention, a device adapted for carrying out a method of the invention is provided comprising
a) an analyzer unit comprising a detection agent which specifically binds to a BNP type peptide, and a detection agent which specifically binds a cardiac troponin, said unit being adapted for determining the amounts of the markers in a sample from a subject suffering from heart failure; and
b) an analyzer unit for comparing the determined amounts with reference amounts, whereby heart failure treatment is guided, said unit comprising a database with reference amounts, and a algorithm for carrying out the comparison.

Preferably, the algorithms are computer-implemented.

Preferred reference amounts and algorithms are disclosed elsewhere herein.

A preferred embodiment of the instant disclosure includes a system for guiding heart failure treatment in a subject suffering from heart failure. Examples of systems include clinical chemistry analyzers, coagulation chemistry analyzers, immunochemistry analyzers, urine analyzers, nucleic acid analyzers, used to detect the result of chemical or biological reactions or to monitor the progress of chemical or biological reactions. More specifically, exemplary systems of the instant disclosure may include Roche Elecsys™ Systems and Cobas® e Immunoassay Analyzers, Abbott Architect™ and Axsym™ Analyzers, Siemens Centaur™ and Immulite™ Analyzers, and Beckman Coulter UniCel™ and Acess™ Analyzers, or the like.

Embodiments of the system may include one or more analyzer units utilized for practicing the subject disclosure. The analyzer units of the system disclosed herein are in operable communication with the computing device disclosed herein through any of a wired connection, Bluetooth, LANS, or wireless signal, as are known. Additionally, according to the instant disclosure, an analyzer unit may comprise a stand-alone apparatus, or module within a larger instrument, which performs one or both of the detection, e.g. qualitative and/or quantitative evaluation of samples for diagnostic purpose. For example, an analyzer unit may perform or assist with the pipetting, dosing, mixing of samples and/or reagents. An analyzer unit may comprise a reagent holding unit for holding reagents to perform the assays. Reagents may be arranged for example in the form of containers or cassettes containing individual reagents or group of reagents, placed in appropriate receptacles or positions within a storage compartment or conveyor. Detection reagents may also be in immobilized form on a solid support which are contacted with the sample. Further, an analyzer unit may include a process and/or detection component which is optimizable for specific analysis.

According to some embodiments, an analyzer unit may be configured for optical detection of an analyte, for example a marker, with a sample. An exemplary analyzer unit configured for optical detection comprises a device configured for converting electro-magnetic energy into an electrical signal, which includes both single and multi-element or array optical detectors. According to the present disclosure, an optical detector is capable of monitoring an optical electro-magnetic signal and providing an electrical outlet signal or response signal relative to a baseline signal indicative of the presence and/or concentration of an analyte in a sample being located in an optical path. Such devices may also include, for example, photodiodes, including avalanche photodiodes, phototransistors, photoconductive detectors, linear sensor arrays, CCD detectors, CMOS detectors, including CMOS array detectors, photomultipliers, and photomultiplier arrays. According to certain embodiments, an optical detector, such as a photodiode or photomultiplier, may contain additional signal conditioning or processing electronics. For example, an optical detector may include at least one pre-amplifier, electronic filter, or integrated circuit. Suitable pre-preamplifiers include, for example, integrating, transimpedance, and current gain (current mirror) preamplifiers.

Additionally, one or more analyzer unit according to the instant disclosure may comprise a light source for emitting light. For example, a light source of an analyzer unit may consist of at least one light emitting element (such as a light emitting diode, an electric powered radiation source such as an incandescent lamp, an electroluminescent lamp, a gas discharge lamp, a high-intensity discharge lamp, a laser) for measuring analyte concentrations with a sample being tested or for enabling an energy transfer (for example, through florescent resonance energy transfer or catalyzing an enzyme).

Further, an analyzer unit of the system may include one or more incubation units (for example, for maintaining a sample or a reagent at a specified temperature or temperature range). In some embodiments, an analyzer unit may include a thermocycler, include a real-time thermocycler, for subjecting a sample to repeated temperature cycles and monitoring a change in the amount of an amplification product with the sample.

Additionally, an analyzer unit of the system disclosed herein may comprise, or be operationally connected to, a reaction vessel or cuvette feeding unit. Exemplary feeding units include liquid processing units, such as a pipetting unit, to deliver samples and/or reagents to the reaction vessels. The pipetting unit may comprise a reusable washable needle, e.g. a steel needle, or disposable pipette tips. The analyzer unit may further comprise one or more mixing units, for example a shaker to shake a cuvette comprising a liquid, or a mixing paddle to mix liquids in a cuvette, or reagent container.

It follows from the above that according to some embodiments of the instant disclosure, portions of some steps of methods disclosed and described herein may be performed by a computing device. A computing device may be a general purpose computer or a portable computing device, for example. It should also be understood that multiple computing devices may be used together, such as over a network or other methods of transferring data, for performing one or more steps of the methods disclosed herein. Exemplary computing devices include desktop computers, laptop computers, personal data assistants ("PDA"), such as BLACKBERRY brand devices, cellular devices, tablet computers, servers, and the like. In general, a computing device comprises a processor capable of executing a plurality of instructions (such as a program of software).

A computing device has access to a memory. A memory is a computer readable medium and may comprise a single storage device or multiple storage devices, located either locally with the computing device or accessible to the computing device across a network, for example. Computer-readable media may be any available media that can be accessed by the computing device and includes both volatile and non-volatile media. Further, computer readable-media may be one or both of removable and non-removable media. By way of example, and not limitation, computer-readable media may comprise computer storage media. Exemplary computer storage media includes, but is not limited to, RAM, ROM, EEPROM, flash memory or any other memory technology, CD-ROM, Digital Versatile Disk (DVD) or other optical disk storage, magnetic cassettes, magnetic tape, magnetic disk storage or other magnetic storage devices, or any other medium which can be used for storing a plurality of instructions capable of being accessed by the computing device and executed by the processor of the computing device.

According to embodiments of the instant disclosure, software may include instructions which, when executed by a processor of the computing device, may perform one or more steps of the methods disclosed herein. Some of the instructions may be adapted to produce signals that control operation of other machines and thus may operate through those control signals to transform materials far removed from the computer itself. These descriptions and representations are the means used by those skilled in the art of data processing, for example, to most effectively convey the substance of their work to others skilled in the art.

The plurality of instructions may also comprise an algorithm which is generally conceived to be a self-consistent sequence of steps leading to a desired result. These steps are those requiring physical manipulations of physical quantities. Usually, though not necessarily, these quantities take the form of electrical or magnetic pulses or signals capable of being stored, transferred, transformed, combined, compared, and otherwise manipulated. It proves convenient at times, principally for reasons of common usage, to refer to these signals as values, characters, display data, numbers, or the like as a reference to the physical items or manifestations in which such signals are embodied or expressed. It should be borne in mind, however, that all of these and similar terms are to be associated with the appropriate physical quantities and are merely used here as convenient labels applied to these quantities. According to some embodiments of the instant disclosure, an algorithm for carrying out a comparison between a determined amount of one or more markers disclosed herein, and a suitable reference, is embodied and performed by executing the instructions. The results may be given as output of parametric diagnostic raw data or as absolute or relative amounts. According to various embodiments of the system disclosed herein, a "diagnosis" may be provided by the computing device of a system disclosed herein based on said comparison of the calculated "amount" to a reference or a threshold. For example, a computing device of a system may provide an indicator, in the form of a word, symbol, or numerical value which is indicative of a particular diagnosis.

The computing device may also have access to an output device. Exemplary output devices include fax machines, displays, printers, and files, for example. According to some embodiments of the present disclosure, a computing device may perform one or more steps of a method disclosed herein, and thereafter provide an output, via an output device, relating to a result, indication, ratio or other factor of the method.

Finally, the invention pertains to a kit adapted for carrying out a method of the present invention comprising a detection agent which specifically binds to a cardiac troponin, and a detection agent which specifically binds to a BNP-type peptide, reference standards as well as instructions for carrying out the said method.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a optical storage medium (e.g., a Compact Disc) or directly on a computer or data processing device. Further, the kit shall comprise at least one standard for a reference as defined herein above, i.e. a solution with a pre-defined amount for the SP-B peptide polypeptide representing a reference amount. Such a standard may represent, e.g., the amount of SP-B peptide from a subject or group of subjects exhibiting a symptom of an acute cardiovascular event and suffering from a pulmonary complication or a subject or group of subjects exhibiting a symptom of an acute cardiovascular event and not suffering from a pulmonary complication or a clinically apparently healthy subject or group thereof.

In some embodiments, a kit disclosed herein includes at least one component or a packaged combination of components for practicing a disclosed method. By "packaged combination" it is meant that the kits provide a single package that contains a combination of one or more components, such as probes (for example, an antibody), controls, buffers, reagents (for example, conjugate and/or substrate) instructions, and the like, as disclosed herein. A kit containing a single container is also included within the definition of "packaged combination." In some embodiments, the kits include at least one probe, for example an antibody (having specific affinity for an epitope of a biomarker as disclosed herein. For example, the kits may include an antibody that is labelled with a fluorophore or an antibody that is a member of a fusion protein. In the kit, the probe may be immobilized, and may be immobilised in a specific conformation. For example, an immobilized probe may be provided in a kit to specifically bind target protein, to detect target protein in a sample, and/or to remove target protein from a sample.

According to some embodiments, kits include at least one probe, which may be immobilized, in at least one container. Kits may also include multiple probes, optionally immobilized, in one or more containers. For example, the multiple probes may be present in a single container or in separate containers, for example, wherein each container contains a single probe.

In some embodiments, a kit may include one or more non-immobilized probe and one or more solid support that does or does not include an immobilized probe. Some such embodiments may comprise some or all of the reagents and supplies needed for immobilizing one or more probes to the solid support, or some or all of the reagents and supplies needed for binding of immobilized probes to specific proteins within a sample.

In certain embodiments, a single probe (including multiple copies of the same probe) may be immobilized on a single solid support and provided in a single container. In other embodiments, two or more probes, each specific for a different target protein or a different form of a single target protein (such as a specific epitope), a provided in a single container. In some such embodiments, an immobilized probe may be provided in multiple different containers (e.g., in single-use form), or multiple immobilized probes may be provided in multiple different containers. In further embodiments, the probes may be immobilized on multiple different type of solid supports. Any combination of immobilized probe(s) and container(s) is contemplated for the kits disclosed herein, and any combination thereof may be selected to achieve a suitable kit for a desired use.

A container of the kits may be any container that is suitable for packaging and/or containing one or more components disclosed herein, including for example probes (for example, an antibody), controls, buffers, and reagents (for example, conjugate and/or substrate). Suitable materials include, but are not limited to, glass, plastic, cardboard or other paper product, wood, metal, and any alloy thereof. In some embodiments, the container may completely encase an immobilized probe(s) or may simply cover the probe to minimize contamination by dust, oils, etc., and expose to light. In some further embodiments, he kits may comprise a single container or multiple containers, and where multiple containers are present, each container may be the same as all other containers, different than others, or different than some but not all other containers.

The Figures show:

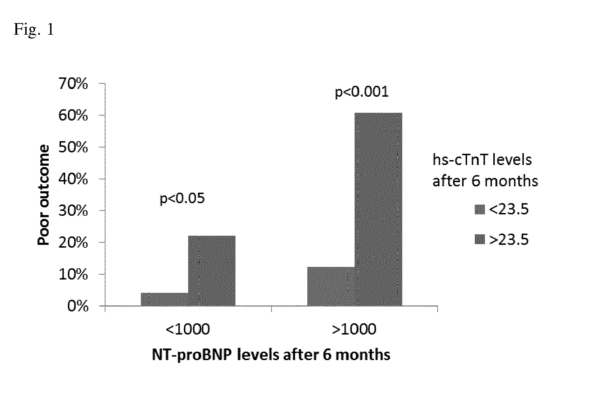

- **Fig. 1:**: Poor outcome (defined as death or repeated or prolonged [i.e. > 14 days] hospitalization due to heart failure; good outcome survival > 3 years without heart failure hospitalization and no prolonged hospitalizations due to other causes. Case-control from the original TIME-CHF population, n=194) after the initial 6 months in TIME-CHF where medical therapy was intensified in patients with NT-proBNP Ievels below and above 1000 pg/mL showing that hs-cTnT levels measured at the same time-point significantly adds to prediction of risk.

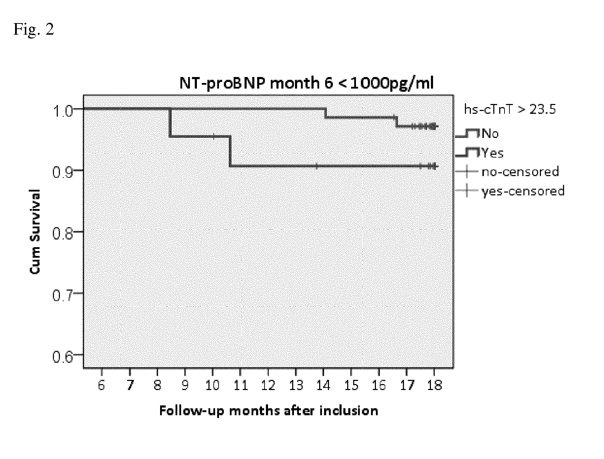

- **Fig. 2:**: Survival after 6 months, NT-proBNP below 1000 pg/ml

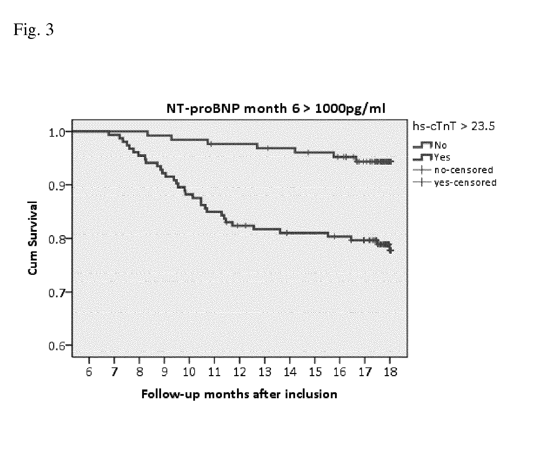

- **Fig. 3:**: Survival after 6 months, NT-proBNP above 1000 pg/ml

All references referred to above are herewith incorporated by reference with respect to their entire disclosure content as well as their specific disclosure content explicitly referred to in the above description.

### EXAMPLES

The invention will now be illustrated by the following Examples which are not intended to restrict or limit the scope of this invention.

### Example 1: Assays

Troponin T was determined in plasma samples using Roche's electrochemiluminescence ELISA sandwich test Elecsys Troponin T hs (high sensitive) STAT (Short Turn Around Time) assay. The test employs two monoclonal antibodies specifically directed against human cardiac troponin T. The antibodies recognize two epitopes (amino acid position 125-131 and 136-147) located in the central part of the cardiac troponin T protein, which consists of 288 amino acids. The hs-TnT assay allows a measurement of troponin T levels in the range of 3 to 10000 pg/mL.

NT-proBNP was determined in plasma samples using Roche's electrochemiluminescence ELISA sandwich test Elecsys proBNP II STAT (Short Turn Around Time) assay. The test employs two monoclonal antibodies which recognize epitopes located in the N-terminal part (1-76) ofproBNP (1-108).

### Example 2: Patient cohort/Results

Patients included in this study were randomized to either NT-proBNP guided or symptom guided heart failure therapy. Uptitration based in study intervention was done within 6 months and patients were followed-up for another 12 months. Long-term results on mortality and hospitalisation are also available.

It was tested if poor outcome is different in patients below or above NT-proBNP target. In patients with NT-proBNP levels below target and poor outcome, it was tested whether cTnT-hs could be helpful as additional marker to identify patients at risk and to guide heart failure therapy.

499 patients suffering from HF (NYHA class II-IV systolic HF (LVEF ≤45%) were guided according to NT-proBNP target or usual care (Pfisterer M. et al. JAMA. 2009; 301:383-92). Overall, patients with NT-proBNP levels <1000 pg/ml, a previously identified cut-off value for good outcome, had significantly better outcome than those with NT-proBNP levels that could not be reduced to these levels. However, some of the patients with low NT-proBNP levels < 1000 pg/mL remained at risk. This risk could be identified with good accuracy by additionally measure cTnT levels, for example after 6 months of therapy. Moreover, cTnT-hs also provided additional important information for potential therapy guidance in the group with higher risk (i.e. NT-proBNP levels after 6 months >1000pg/ml). Thus, in this group patients with excessive risk could be identified requiring immediate action, whereas in the group with NT-proBNP >1000pg/ml, but low cTnT-hs the risk was even smaller than in those with NT-proBNP <1000pg/ml but elevated cTnT-hs of >23.5pg/ml.

### Example 3: Case Studies

A 78 year old male patient with class C heart failure is receiving furosemide, enalapril, and metoprolol at standard recommended doses. The patient does not show signs or symptoms of worsening HF at the regular visit. NT-ProBNP and Troponin T are determined in a plasma sample obtained from the patient. The NT-proBNP value is below 1000 pg/mL and the Troponin T value is below 23.5 pg/ml. The therapy is maintained and the patient remains stable with a good outcome until the end of the study (no death or hospitalization).
•A 83 year old male patient with class D heart failure is receiving i.v. furosemide, perindopril, digoxin, and atenololol at maximally recommended doses. He also has an implantable defibrillator device (ICD). The patient has had episode of decompensation and hospitalization in the past, but has been stable for the past 3 months. NT-ProBNP and Troponin T are determined in a plasma sample obtained from the patient. The NT-proBNP value is above 1000 pg/mL and the Troponin T value is above 23.5 pg/ml. The therapy is intensified, i.e. spironolactone is added at the recommended starting dose. Two weeks later, both NT-proBNP and cTnT-hs are decreased but not below target levels. The dose of spironolactone is uptitrated and a combined defibrillator / cardiac resynchronization stimulator implanted over the course of 12 weeks until NT-proBNP value falls below 1000 pg/mL and the Troponin T value falls below 23.5 pg/ml. Thereafter the patient remains stable with a relatively good outcome until the end of the study (no death, one short hospitalization for dyspnea with discharge on the same day).
•A 68 year old female patient with class C heart failure is receiving captopril at standard therapeutic doses. The patient has been stable for 9 months after a previous hospitalization for acute heart failure. NT-ProBNP and Troponin T are determined in a plasma sample obtained from the patient at a pre-scheduled visit. The NT-proBNP value is below 1000 pg/mL and the Troponin T value is above 23.5 pg/ml. The therapy is maintained and the patient returns home. Three weeks later, the patient is hospitalized for acute dyspnea with atrial fibrillation, pulmonary congestion and gradual worsening of the cardiac function over the course of the next 14 days, resulting in transfer to the Cardiac Care Unit where the patient dies after 5 days due to pump failure. This patient was at increased risk of heart failure decompensation that was not evident based on the clinical presentation and NT-proBNP value alone. However, the cTnT-hs indicated the elevated risk and therapy should have been intensified at the pre-scheduled visit. Addition of a beta blocker such as metoprolol and an aldosterone antagonist such as spironolactone would have been a beneficial medication and could have prevented the poor outcome.

### Conclusions

The present invention provides a method combining a BNP-type peptide and of a cardiac Troponin for monitoring and guiding therapy in heart failure patients. Compared to prior art and to previous biomarker guided heart failure approaches, the invention provides both cTnT and NT-proBNP target levels. The invention improves the identification of patients at risk of adverse events that would otherwise not benefit from guided heart failure therapy done by a BNP-type peptide alone. Thus, the invention aims at reducing the mortality and morbidity in heart failure patients.

## Claims

1. A method for guiding heart failure treatment in a subject suffering from heart failure, said method comprising the steps of
a) determining the amount of a BNP-type peptide and of a cardiac Troponin in a sample from the subject, and
b) comparing the amount of the BNP-type peptide to a reference amount for the BNP-type peptide, and the amount of the cardiac Troponin to a reference amount for the cardiac Troponin, whereby heart failure treatment is guided.

2. The method of claim 1, wherein the heart failure treatment comprises administration of at least one medicament selected from the group consisting of diuretics, angiotensin converting enzyme inhibitors, angiotensin II receptor blockers, beta blockers and aldosterone antagonists, in particular wherein the heart failure treatment comprises combined administration of a beta blocker and an ACE inhibitor.

3. The method of claim 1 or 2, wherein the subject is human.

4. The method of any one of claims 1 to 3, wherein the subject did not experience worsening of heart failure symptoms prior to obtaining the sample, in particular wherein the subject did not experience worsening of heart failure symptoms within a period of three months prior to obtaining the sample.

5. The method of any one of claims 1 to 3, wherein the sample is a blood, serum or plasma sample.

6. The method of any one of claims 1 to 3, wherein the BNP-type peptide is BNP or NT-proBNP, and/or wherein the cardiac Troponin is Troponin T.

7. The method of any one of claims 1 to 5, wherein the reference amount for the cardiac Troponin is an amount of about 23.5 pg/ml, if the cardiac Troponin is Troponin T, wherein the reference amount for the BNP-type peptide is about 100 pg/ml if the BNP-type peptide is BNP, and/or wherein the reference amount for the BNP-type peptide is about 1000 pg/ml if the BNP-type peptide is NT-proBNP.

8. The method according to any one of claims 1 to 7, wherein
i) an amount of the cardiac Troponin larger than the reference amount for the cardiac Troponin, and/or an amount of the BNP-type peptide larger than the reference amount for the BNP-type peptide indicates that the subject requires intensification of the heart failure treatment, and/or
ii) an amount of the cardiac Troponin lower than the reference amount for the cardiac Troponin, and an amount of the BNP-type peptide lower than the reference amount for the BNP-type peptide indicates that the subject does not require intensification of the heart failure treatment.

9. The method of claim 8, wherein the heart failure treatment intensification comprises increasing the dosage of previously administered medicaments, the administration of a further medicament (or medicaments), in particular the administration of a further medicament (or medicaments) having a different mode of action that the previously administered medicaments, device therapy, life style changes, and combinations thereof.

10. The method of any one claims 1 to 9, wherein the subject is a subject who requires heart failure treatment intensification, wherein the method further comprises the steps of
c) determining the amount of a BNP-type peptide and of a cardiac Troponin in a further sample from the subject, and
d) comparing the amount of the BNP-type peptide to a reference amount for the BNP-type peptide, and the amount of the cardiac Troponin to a reference amount for the cardiac Troponin,
wherein the further sample has been obtained between two weeks and three months after the sample of step a).

11. The method of any one claims 1 to 9, wherein the subject is a subject who does not require heart failure treatment intensification, wherein the method further comprises the steps of
c) determining the amount of a BNP-type peptide and of a cardiac Troponin in a further sample from the subject, and
d) comparing the amount of the BNP-type peptide to a reference amount for the BNP-type peptide, and the amount of the cardiac Troponin to a reference amount for the cardiac Troponin,
wherein the further sample has been obtained between three months and twelve months after the sample of step a).

12. The method of any one of claims 1 to 11, wherein the method does not encompass the determination of the amount of GDF-15 (Growth Differentiation Factor 15).

13. Use of i) of a BNP-type peptide and of a cardiac Troponin or of ii) a detection agent which specifically binds to a BNP-type peptide, and of a detection agent which specifically binds to a cardiac Troponin, in a sample from a subject suffering from heart failure for guiding heart failure treatment.

14. A device adapted for carrying out a method of any one of claim 1 to 12 comprising
a) an analyzer unit comprising a detection agent which specifically binds to a BNP type peptide, and a detection agent which specifically binds a cardiac troponin, said unit being adapted for determining the amounts of the markers in a sample from a subject suffering from heart failure; and
b) an analyzer unit for comparing the determined amounts with reference amounts, whereby heart failure treatment is guided, said unit comprising a database with reference amounts as set forth in claim 7, and a algorithm as set forth in claim 8 for carrying out the comparison.
